# EUROPÄISCHE PATENTSCHRIFT

(11) **EP 1 537 423 B1**
(45) Veröffentlichungstag und Bekanntmachung des Hinweises auf die Patenterteilung: **31.05.2006**
(21) Anmeldenummer: 03794993.0
(22) Anmeldetag: 04.09.2003
(51) Int. Cl.: G01N 33/88, G01N 33/68

(54) **VERFAHREN ZUM LOKALEN NACHWEIS VON ENTZÜNDUNGS- UND ALLERGIE-MEDIATOREN**
METHOD FOR THE LOCAL DETECTION OF INFLAMMATION AND ALLERGY MEDIATORS
PROCEDE DE DETECTION LOCALE DE MEDIATEURS D'INFLAMMATION ET D'ALLERGIE

(30) Priorität: 09.09.2002 DE 10241700; 09.09.2002 US 409287 P
(43) Veröffentlichungstag der Anmeldung: 08.06.2005
(73) Patentinhaber: Medifact-Publishing GmbH, 81475 München (DE)
(72) Erfinder: WEISSENBACHER, Ernst, Rainer, 81475 München (DE)
(74) Vertreter: von Samson-Himmelstjerna, Friedrich
(86) Internationale Anmeldenummer: PCT/EP2003/009841
(87) Internationale Veröffentlichungsnummer: WO 2004/025304

(56) Entgegenhaltungen:
- EP-A- 0 687 910
- WO-A-02/22803
- WO-A-02/092826
- US-B1- 6 190 872
- GASPARD I ET AL: "Quantitation of Cytokine mRNA Expression as an Endpoint for Prediction and Diagnosis of Xenobiotic-Induced Hypersensitivity Reactions" METHODS: A COMPANION TO METHODS IN ENZYMOLOGY, ACADEMIC PRESS INC., NEW YORK, NY, US, Bd. 19, Nr. 1, September 1999 (1999-09), Seiten 64-70, XP004466806 ISSN: 1046-2023
- MORI JUNICHI ET AL: "Cytokine mRNA expression in vernal keratoconjunctivitis" NIPPON GANKA GAKKAI ZASSHI, Bd. 106, Nr. 7, Juli 2002 (2002-07), Seiten 392-397, XP009022609 ISSN: 0029-0203
- WITKIN S S ET AL: "A localized vaginal allergic response in women with recurrent vaginitis." THE JOURNAL OF ALLERGY AND CLINICAL IMMUNOLOGY. UNITED STATES FEB 1988, Bd. 81, Nr. 2, Februar 1988 (1988-02), Seiten 412-416, XP009022944 ISSN: 0091-6749

## Beschreibung

Die vorliegende Erfindung betrifft ein qualitatives oder quantitatives Nachweisverfahren für das lokale Vorliegen von Entzündungs- und Allergie-Mediatoren, wie Cytokinen, Prostaglandinen und Leukotrienen.

Sehr viele Frauen leiden an akuten oder chronischen Vaginalinfektionen, die häufig von Juckreiz und Schmerzen und gegebenenfalls Ausflüss begleitet sind. Einer der häufigsten Erreger dieser Infektionen ist *Candida albicans,* und über 75% aller Frauen hatten ein oder mehrere Male eine Infektion mit diesem Erreger.

*C. albicans* ist eine pleiomorphe Hefe, die sowohl in der Hyphen- als auch in der Myzel- (echtes oder Pseudo-) Form auftritt. Die Hyphenform verursacht, im Gegensatz zu der Myzelform, keine klinischen Symptome. Der Nachweis von *C*. *albicans* geschieht in Kultur eines Vaginalabstrichs.

Candida-Infektionen treten in asymptomatischer, akuter, persistenter, wiederkehrender oder chronisch wiederkeh-Candida-Infektionen treten in asymptomatischer, akuter, persistenter, wiederkehrender oder chronisch wiederkehrender Form auf. Die asymptomatische Form ist nicht immer in einer Kultur nachweisbar. Die Äthiologie der chronisch wiederkehrenden Candidiasis nach einem Zeitraum mit negativer Hefekultur ist nicht eindeutig geklärt. Es wird angenommen, daß es sich um einen zellulären Immundefekt handelt, da T-Zellen und Makrophagen in ungenügender Zahl vorliegen. Möglicherweise spielt auch eine allergische Reaktion auf das Candida-Allergen eine Rolle, welche die zelluläre Immunabwehr unterdrückt.

Eine weiterer potentieller Erreger für Vaginalinfektionen ist *Escherichia choli. E. choli* ist ein allgegenwärtiges Bakterium, das natürlich in der Darmflora vorkommt. Man kann jedoch zahlreiche Serotypen unterscheiden, von denen einige pathogen sind und beispielsweise eine Kolpitis und/oder Harnwegsinfektion hervorrufen können. Der Nachweis von *E*. *choli* findet gewöhnlich ebenfalls in Kultur statt. Dabei ist es jedoch schwierig, zwischen pathogenen und nicht-pathogenen Stämmen zu unterscheiden.

Die beiden oben genannten Infektionen sind lediglich Beispiel für besonders häufige Vaginalinfektionen, und der Fachmann weiß, daß es zahlreiche weitere Beispiele dafür gibt.

Eine frühzeitige Erkennung und Behandlung von Vaginalinfektionen ist bei einer bestehenden Schwangerschaft besonders wichtig, da sie zu einer Frühgeburt führen können.

Es wäre also wünschenswert, über das klassische Verfahren einer Erreger-Kultur hinaus über ein schnelles und einfach durchzuführendes Verfahren zu verfügen, das Auskunft gibt, ob eine entzündliche und/oder allergische Reaktion in der Scheide vorliegt. Dies hat sich die vorliegende Erfindung zur Aufgabe gestellt.

Demgemäß betrifft die vorliegende Erfindung ein Verfahren zum lokalen qualitativen oder quantitativen Nachweis von Entzündungs- und/oder Allergie-Mediatoren mittels PCR (Polymerase Chain Reaction), LCR (Ligase Chain Reaction) und/oder Enzym- oder Radioimmunoassay in einer Probe, das dadurch gekennzeichnet ist, daß die Probe ein Vaginalabstrich-, -sekret oder eine Vaginallavage ist.

Bei den Entzündungs- und/oder Allergie-Mediatoren, die erfindungsgemäß nachgewiesen werden, handelt es sich insbesondere um Cytokine, wie Tumornekrosefaktor (TNF), Interferon-γ (IFN-γ) und die Interleukine (IL), beispielsweise IL-1, IL-2, IL-4, IL-6, IL-10 and IL-12 und der IL-1-Rezeptorantagonist (IL-1ra), und um Lipid-Mediatoren, wie die Prostaglandine (z*.*B*.* PGE₁, PGE₂, PGI₂ und PGF₂) und Leuktriene (z.B. LTB₄).

Bei der Probe handelt es sich bevorzugt um denselben Vaginalabstrich, dasselbe Vaginalsekret oder dieselbe Vaginallavage, der bzw. das bzw. die für die Zytologie und den Nachweis von pathogenen Erregern verwendet wird.

Cytokine können grob in zwei Klassen eingeteilt werden:
(1) entzündungsfördernde Cytokine; dazu gehören TNF, IFN-γ, IL-1, IL-2 und I1-12; sie stehen mit der T-Zell-vermittelten Immunantwort in Beziehung, z.B. der Differenzierung von CD4⁺-Zellen zu T_{H}1-Zellen und der Aktivierung von Makrophagen und NK-Zellen, und hemmen die Bildung von T_{H}2-Zellen.
(2) entzündungshemmende Cytokine: dazu gehören IL-4, IL-10 und IL-1ra, der ein natürlicher kompetitiver Inhibitor von IL-1 ist, um eine überschießende IL-1-Produktion zu verhindern. IL-4 stimuliert die Differenzierung von CD4⁺-Zellen zu T_{H}2-Zellen und wird, zusammen mit IL-10, wiederum von diesen sekretiert. IL-4 und IL-10 hemmen die Makrophagen-Aktivierung und die Differenzierung von CD4⁺-Zellen zu T_{H}1-Zellen. IL-4 ist das Haupt-Cytokin, das die Produktion von IgE und damit eine allergische Reaktion stimuliert.

IL-6 nimmt eine Zwischenstellung zwischen diesen beiden Klassen ein.

Viren und Bakterien, die intrazellulär leben, stimulieren eine zelluläre Immunantwort unter Beteiligung der unter (1) aufgeführten Cytokine. Gewöhnliche Bakterien stimulieren meist sowohl die T_{H}1-Zellenbildung als auch die T_{H}2-Zellenbildung. Es liegen dann sowohl die unter (1) als auch die unter (2) aufgeführten Cytokine vor.

Die herrschende Meinung ist, daß auch für mit *C*. *albicans* infizierter Schleimhaut die zelluläre Immunantwort entscheidend ist, d.h. die unter (1) aufgeführten Cytokine werden bei einer derartigen Infektion vorgefunden. Wenn dies, trotz positiver Kultur, nicht oder nur in geringem Ausmaß der Fall ist und vor allem die unter (2) angeführten Cytokine nachgewiesen werden, liegt eine Allergie gegen *Candida* vor, was zur Folge hat, daß wegen der Unterdrückung der T_{H}1-Zellbildung keine zellvermittelte Immunantwort zustande kommt. Dies könnte eine der Ursachen für die (ggf. chronisch) wiederkehrende Infektion sein, wenn bei der Behandlung nicht alle Keime getötet worden sind.

Insbesondere die Anwesenheit der unter (1) aufgeführten Cytokine in einer untersuchten Vaginalabstrich-, -sekret-, oder -lavage-Probe gestatten den Schluß, daß ein pathogener Keim vorhanden ist. Ihr Fehlen und das Vorhandensein von der unter (2) aufgeführten Cytokine kann, wenn durch Kultur die Anwesenheit eines solchen Keims nachgewiesen ist, den Schluß zulassen, daß ein Immundefekt und/oder eine allergische Reaktion vorliegt.

Auch Prostaglandine und Leukotriene sind bedeutende Entzündungsmediatoren. Sie weisen eine lokal und zeitlich begrenzte Wirkung an ihrem Wirkort, z.B. dem Ort der Entzündung, auf. PGE₂ führt zu Vasodilatation und potenziert die erhöhte vaskuläre Permeabilität, die von Histamin und Bradykinin erzeugt wird. LTB₄ wird ebenfalls am Ort der Entzündung gebildet und ruft u.a. eine chemotaktische Reaktion hervor, bei der Leukozyten zum Ort der höchsten LZB₄-Konzentration wandern. Ihr Nachweis im Vaginalabstrich deutet ebenfalls auf eine Entzündung hin.

Der Nachweis der Cytokine, bei den es sich ja um Peptide handelt, kann mittels PCR oder LCR oder auch anhand eines ELISA (enzyme linked immunosorbent assay) vorgenommen werden.
Geeignete ELISA-Kits für Cytokine sind z.B. von der Firma BioSource International erhältlich.

Bei der PCR oder LCR wird gewöhnlich zunächst eine Reverse Transcription (RT) der vorhandenen mRNA in cDNA vorgenommen. Diese cDNA wird dann unter Verwendung eines Cytokin-spezifischen Primerpaars mittels PCR oder LCR auf solche Weise amplifiziert, daß eine qualitative oder vorzugsweise quantitative Bestimmung möglich ist.

Bei der PCR geschieht dies z.B. mit Hilfe einer Sonde, die in geeignetem Abstand für ein Quenchen gemäß dem Förstermechanismus eine fluoreszierende Markierung und einen deren Fluoreszenz quenchenden Fluoreszenzquencher aufweist und mit der gesuchten Cytokinsequenz hybridisiert. Bei der Hybridisierung - und nur dann - spaltet die Taq-Polymerase die Sonde zwischen dem Fluoreszenzfarbstoff und dem Fluoreszenzquencher. Aufgrund des vergrößerten Abstands zwischen diesen findet dann kein Fluoreszenzquenchen mehr statt. Die nun auftretenden Fluoreszenz ist, wenn sie geeignet anhand eines Standards normalisiert wird, ein direktes Maß für die Größe des Signals. Aus diesem kann bei geeigneter Kalibirierung die anfängliche Zahl der Kopien der gesuchten Sequenz bestimmt werden.

Ein geeignetes Kit zur Durchführung einer solchen PCR für diverse Interleukine mit den entsprechenden Primerpaaren und Sonden ist von der Firma BioSource International, Inc. als Real Time PCR® Reagents auf dem Markt. Diese Reagenzien sind zur Verwendung mit dem Taq-Reagenzkit ABI TaqMan® Universal PCR Master Mix Reagents optimiert, können aber auch mit anderen Taq-Reagenzien verwendet werden. Reverse Transcriptions-Reagenzien sind z.B. von Applied Biosystems (ABI), Stratagene, Clontech erhältlich.

Prostaglandine und Leukotriene werden bevorzugt mittels ELISA quantitativ bestimmt. Geeignete Antikörper sind von einer Vielfalt von Quellen, u.a. BioSource International Inc. und der Acris GmbH, D-32120 Hiddenhausen, erhältlich, die auch komplette ELISA-Kits für die wichtigsten Prostaglandine und für LTB₄ liefert.

Auf Grund der Empfindlichkeit dieser beiden Techniken ist es möglich, mit einem sehr geringen Probenvolumen auszukommen. Demgemäß kann ein einziger Abstrich dazu verwendet werden, eine zytologische Untersuchung, Nachweise von Keimen und die Nachweise von Entzündungsmediatoren durchzuführen.

Die folgenden Beispiele erläutern die Erfindung, ohne sie zu beschränken.

### Beispiel 1

### Bestimmung von Interleukienen

Zur Entnahme von Vaginalzell- und -sekretmaterial werden mittels einer sterilen Spritze 2 ml sterile physiologische Kochsalzlösung in die Scheide eingebracht. Mittels eines Watteträgers wird eine Vaginallavage vorgenommen. Mit einer sterilen Spritze wird dann 1 ml der so erhaltenen Lavageflüssigkeit entnommen und sofort ins Labor gebracht.

Dort wird die Flüssigkeit 2 min bei 8000 U/min zentrifugiert, und der Überstand wird bei -70°C aufbewahrt. Das Pellet wird für die zytologische und mikrobielle Untersuchung sowie für gegebenenfalls für eine PCR verwendet.

### 1.1 ELISA von IL-10

Für den ELISA wird der Überstand aufgetaut und bei 4°C gehalten.

Der ELISA wird mittels eines kommerziellen Kits (BioSource International, Katalog-Nr.KHC0101) bei 4°C durchgeführt.

### 1.2 RT-PCR mit IL-2

Gemäß den üblichen Verfahren wurde das Pellet aus der Zentrifugation lysiert und wurden die Nukleinsäuren gewonnen.

Mit der so erhaltenen Probe wurde zunächst eine Reverse Transcription mit dem RT-Kit TaqMan® Reverse Transcription Reagents (ABI) gemäß den Anweisungen des Herstellers durchgeführt.

Dann wurden unter Verwendung einer IL-2 FRET-Sonde (ABI), eines humanen IL-2-Primerpaars (ABI), des ABI TaqMan® Universal Master Mix und der oben erzeugten cDNA sowie einer Blindprobe, einer positiven Kontrolle und einer RNA-Kontrolle Reaktionsmischungen gemäß den Empfehlungen des Herstellers für die PCR hergestellt. Die empfohlenen Mengen (jeweils 50 µl) wurden in Röhrchen überführt und zum Mischen und Entfernen von Luftblasen zentrifugiert. Dann wurde jede Reaktionsmischung in Vertiefungen einer optischen Reaktionsplatte mit 96 Vertiefungen gegeben. Diese wurden verschlossen, und die Platte wurde zentrifugiert, um den Inhalt nach unten zu befördern und Luftblasen zu entfernen.

Die Platten wurden dann in ein ABI PRISM 7700 Sequence Detection System gegeben und wie folgt thermischen Zyklen unterzogen:
Enzym-Aktivierung: 95°C, 10 min;
Thermozyklen (40):
   Denaturierung: 95°C, 15 s
   Hybridisierung, Verlängerung: 60°C, 1 min.

Nach jedem Zyklus wird das Fluoreszenz-Signal gemessen, das in einem Amplifikations-Diagramm wiedergegeben wird. Durch Vergleich mit einer Kalibrierungskurve kann die Zahl der anfänglich vorhandenen Kopien von IL-2 bestimmt werden.

### Beispiel 2

Derselbe Überstand wie oben wurde auch zur Durchführung eines ELISA (enzyme linked immunosorbent assay) für die Bestimmung von LTB₄ verwendet.

Zur Durchführung des Assays wurde das ELISA-Kit für Leukotrien B4 (LTB₄) der Acris GmbH verwendet, dessen Nachweisbereich im Bereich von 8 bis 2000 pg/ml liegt. Es wurde nach den Empfehlungen des Herstellers vorgegangen. Die Anweisungen des Herstellers können im Internet unter der Web-Adresse www.acris-antibodies.de, Katalog-Nr. 900-068, eingesehen werden.

## Patentansprüche

1. Verfahren zum lokalen qualitativen oder quantitiven Nachweis von Entzündungs- und/oder Allergie-Mediatoren mittels PCR (Polymerase Chain Reaction), LCR (Ligase Chain Reaction) und/oder Enzym- oder Radioimmunoassay in einer Probe, die ein Vaginalabstrich, -sekret oder eine Vaginallavage einer Frau ist,
**dadurch gekennzeichnet, daß**
dieselbe Probe auch für eine zytologische Untersuchung und gegebenenfalls für einen Nachweis eines infektiösen Mikroorganismus verwendet wird.

2. Verfahren nach Anspruch 1, **dadurch gekennzeichnet, daß** die Entzündungs- und/oder Allergie-Mediatoren Cytokine sind.

3. Verfahren nach Anspruch 2, **dadurch gekennzeichnet, daß** es sich bei den Cytokinen um den Tumornekrosefaktor, Interferon-γ, Interleukin-1, Interleukin-1ra, Interleukin-2, Interleukin-4, Interleukin-6, Interleukin-10 und Interleukin-12 handelt.

4. Verfahren nach Anspruch 2 oder 3, **dadurch gekennzeichnet, daß** der Nachweis in einer quantitativen RT-PCR (Reverse Transcription-Polymerase Chain Reaction) besteht.

5. Verfahren nach Anspruch 1, **dadurch gekennzeichnet, daß** es sich bei den Entzündungs- und Allergie-Mediatoren um Prostaglandine und Leukotriene handelt.

6. Verfahren nach Anspruch 5, **dadurch gekennzeichnet, daß** der Nachweis in einem ELISA (enzyme linked immunosorbent assay) besteht.

## Claims

1. Method for local qualitative or quantitative detection of inflammatory mediators and/or allergy mediators by means of PCR (polymerase chain reaction), LCR (ligase chain reaction) and/or enzyme immunoassay or radio immunoassay in a specimen, which is a vaginal smear, vaginal secretion or vaginal lavage of a woman,
**characterised in that**,
the same specimen is also used for a cytological investigation and optionally for a detection of an infectious microorganism.

2. Method according to claim 1,
**characterised in that**
the inflammatory mediators and/or allergy mediators are cytokines.

3. Method according to claim 2,
**characterised in that**
the cytokines are the tumour necrosis factor, interferon-γ, interleukin-1, interleukin-1ra, interleukin-2, interleukin-4, interleukin-6, interleukin-10 and interleukin-12.

4. Method according to claim 2 or 3,
**characterised in that**
the detection consists of a quantitative RT-PCR (reverse transcription-polymerase chain reaction).

5. Method according to claim 1,
**characterised in that**
the inflammatory mediators and allergy mediators are prostaglandins and leukotrienes.

6. Method according to claim 5,
**characterised in that**
the detection consists of an ELISA (enzyme-linked immunosorbent assay).

## Revendications

1. Procédé de détection qualitative ou quantitative locale des médiateurs de l'inflammation et/ou de l'allergie par PCR (réaction en chaîne par polymérase), LCR (réaction en chaîne par ligase) et/ou analyse immunoenzymatique ou radioimmunoanalyse dans un échantillon, qui est un frottis vaginal, une sécrétion vaginale ou un lavage vaginal d'une femme, **caractérisé en ce que** ce même échantillon est aussi utilisé pour une analyse cytologique et, le cas échéant, pour une détection d'un micro-organisme infectieux.

2. Procédé selon la revendication 1, **caractérisé en ce que** les médiateurs de l'inflammation et/ou de l'allergie sont des cytokines.

3. Procédé selon la revendication 2, **caractérisé en ce que** les cytokines sont le facteur de nécrose tumoral, l'interféron-γ, l'interleukine-1, l'interleukine-lra, l'interleukine-2, l'interleukine-4, l'interleukine-6, l'interleukine-10 et l'interleukine-12.

4. Procédé selon la revendication 2 ou 3, **caractérisé en ce que** la détection consiste en une RT-PCR quantitative (réaction en chaîne par polymérase après transcription inverse).

5. Procédé selon la revendication 1, **caractérisé en ce que** les médiateurs de l'inflammation et de l'allergie sont des prostaglandines et des leucotriènes.

6. Procédé selon la revendication 5, **caractérisé en ce que** la détection consiste en une analyse ELISA (immunoabsorption enzymatique).
